# EUROPEAN PATENT APPLICATION

(11) **EP 4 681 676 A1**
(43) Date of publication of application: **21.01.2026**
(21) Application number: 24188500.3
(22) Date of filing: 15.07.2024
(51) Int. Cl.: A61B 34/10, A61B 34/20, A61B 34/30, A61B 34/00, A61B 17/00, A61B 90/00, G06T 7/00

(54) **SUPPORT IN HANDLING AN INTERVENTIONAL DEVICE**

(71) Applicant: Koninklijke Philips N.V., 5656 AG Eindhoven (NL)
(72) Inventor: KESSELS, Angelique Carin Johanna Maria, Eindhoven (NL); HENDRIKS, Bernardus Hendrikus Wilhelmus, Eindhoven (NL); BLIJD, Järl John Paul, 5656AG Eindhoven (NL); ELENBAAS, Thijs, Eindhoven (NL); HELMSTRIJD, Ronald Christiaan, Eindhoven (NL); KONTAXIS, Charis, Eindhoven (NL); VAN RIEL, Sjors, Eindhoven (NL); RUIJTERS, Daniel Simon Anna, Eindhoven (NL); OPHEIJ, Martinus Cornelis, Eindhoven (NL)
(74) Representative: Philips Intellectual Property & Standards

(57) **Abstract**

The present invention relates to using an interventional device. In order to further facilitate handling of inserted devices, a support device (10) for handling of an interventional device is provided that comprises a data input (12), a data processor (14) and an output interface (16). The data input is configured to provide 3D anatomic data (18) of a region of interest comprising a vascular structure, and to provide current spatial device data (20) of an interventional device inserted in the vascular structure. The data processor is configured to analyze a mechanical relation (22) of the interventional device and the vascular structure based on the 3D anatomic data and the spatial device data. The output interface is configured to provide the mechanical relation for a handling of the interventional device.

## Description

### FIELD OF THE INVENTION

The present invention relates to using an interventional device. The invention relates in particular to a support device for handling of an interventional device, to a system for handling of an interventional device inserted into a subject and to a method for supporting a handling of an interventional device.

### BACKGROUND OF THE INVENTION

In the field of medical practice, interventional physicians handle the manipulation of devices inside the arteries of a subject, or inside other hollow anatomic structures. As a training for such tasks, connecting and internalizing the manual manipulation of the device is provided, e.g. with the visual feedback that is provided by X-ray images. With many hours of training, the doctor learns which hand-movement leads to which device-movement at the tip of the device. Next to the manipulation of the device tip, the doctor needs to be aware of the entire pathway of the device. To support physicians, motor supported handling devices are also used. This may provide relief in terms of manually applied operating forces, but to avoid unintentional puncture of e.g. a wall enclosing the lumen, for example a vessel wall, high awareness is still needed when handling the device. As an example, US 2020/155249 A1 provides a contact force sensor at the tip of the device to provide an alert to the user. However, it has been shown that handling of inserted devices still requires constant attention and awareness of operating staff members and can thus become a tedious task.

### SUMMARY OF THE INVENTION

There may therefore be a need to further facilitate handling of inserted devices.

The object of the present invention is solved by the subject-matter of the independent claims; further embodiments are incorporated in the dependent claims. It should be noted that the following described aspects of the invention apply also for the support device for handling of an interventional device, for the system for handling of an interventional device inserted into a subject and for the method for supporting a handling of an interventional device.

According to the present invention, a support device for handling of an interventional device is provided. The support device comprises a data input, a data processor and an output interface. The data input is configured to provide 3D anatomic data of a region of interest comprising a vascular structure, and to provide current spatial device data of an interventional device inserted in the vascular structure. The data processor is configured to analyze a mechanical relation of the interventional device and the vascular structure based on the 3D anatomic data and the spatial device data. The output interface is configured to provide the mechanical relation for a handling of the interventional device.

As an effect, the user can be provided with additional information about a current handling of an inserted device. To retrieve the information based on image analysis provides the advantage that sensors on the device are not required, making the device setup less complex and thus beneficial.

According to an example, the analyzing of the mechanical relation comprises computing i) friction between the interventional device and the anatomy, or ii) tension within the interventional device or a combination thereof. In an option, for the analyzing of the mechanical relation, data is provided comprising mechanical properties of the interventional device. In a further option, for analyzing of the mechanical relation, a tortuosity of the interventional device is determined.

In an example, either of the options i) and ii) is provided, in another example both options are provided in combination.

An example for friction is the built up of tension due to non-movement because of friction forces. The friction may then suddenly be overcome by the user pushing further plus the built up tension. A sudden slip may then take place. By providing information about the current mechanical situation, such stick-and-slip can be minimized or even prevented.

According to an example, the output interface is configured to provide the mechanical relation comprising the output feedback based on the mechanical relation to a user operating the interventional device. In an option, the output of feedback comprises to generate haptic and/or tactile feedback, visual feedback, acoustic feedback or electricity feedback or any combination thereof.

This allows e.g. an intuitive way of informing the user which facilitates the handling of a device.

According to an example, to determine the vascular structure, the data processor is configured to provide anatomical modelling. In an option, for the anatomical modelling, the data processor is configured to generate a 3D vessel model representing the vascular structure. In a further option, to generate the 3D vessel model, the data processor is configured to segment the vascular structure in the provided image data, and to quantify the vascular structure in its dimensions.

In an example, either of the options is provided; in another example both options are provided in combination.

This enables to rely on image data, for example image data provided for other purposes anyway.

According to an example, to provide the 3D anatomic data, the data input is configured to provide a 3D model of the region of interest of the anatomy of the subject. The data processor is configured to register the 3D model to the current spatial device data.

According to an example, to provide the spatial device data, the data processor is configured to identify and extract the interventional device within the vascular structure.

According to an example, to provide the spatial device data, the data input is configured to provide shape data from shape sensing provided by the interventional device.

According to an example, the data input is configured to provide image data as a sequence of images. The data processor is configured to provide the image analysis steps comprising at least the extracting of the interventional device as video-processing for a plurality of the images.

According to an example, the data input is configured to provide additional sensor data relating to a current mechanical relation. The data processor is configured to compare the additional sensor data with the analyzed mechanical relation to determine discrepancy information. The output interface is configured to provide the discrepancy information for an adjustment.

According to the present invention, also a system for handling of an interventional device inserted into a subject is provided. The system comprises a device for providing 3D anatomic data of the subject, a support device according to one of the preceding examples and a feedback device. The device for providing 3D anatomic data of the subject provides 3D anatomic data of a region of interest comprising a vascular structure to the support device. The feedback device is data connected to the support device and is configured to output a feedback to the user based on the mechanical relation.

According to an example, the device for providing 3D anatomic data of the subject is an imaging device. The imaging device is configured to provide image data from X-ray imaging, magnetic resonance imaging or ultrasound imaging or a combination thereof.

According to the present invention, also a method for supporting a handling of an interventional device is provided. The method comprises the following steps: providing 3D anatomic data of a region of interest comprising a vascular structure; providing current spatial device data of an interventional device inserted in the vascular structure; analyzing a mechanical relation of the interventional device and the vascular structure based on the 3D anatomic data and the spatial device data; and providing the mechanical relation for a handling of the interventional device.

According to an aspect, image analysis is used for determining forces on an interventional device. This is provided as feedback to a user operating or handling the device. By retracting device/anatomy related information from the image data, the user is provided with additional information improving handling. For example, the handling is improved due to the additional information since the feedback can be used in an intuitive way. Also safety is improved as the user gains more knowledge about what is actually going on.

For extracting the interventional device within the vascular structure, a shape of the device is extracted.

The invention provides support in handling an interventional device.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a support device for handling of an interventional device.
Fig. 2 shows an example of a system for handling of an interventional device inserted into a subject.
Fig. 3 shows an illustration of another example of the system for handling of an interventional device.
Fig. 4 shows an illustration of a further example of the system for handling of an interventional device inserted into a subject in the context of an operation room equipped with an X-ray imaging system.
Fig. 5 shows basic steps of an example of a method for supporting a handling of an interventional device.
Fig. 6 shows a working scheme of another example.
Fig. 7 shows a working scheme of a further example.
Fig. 8 shows a working scheme of a still further example.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

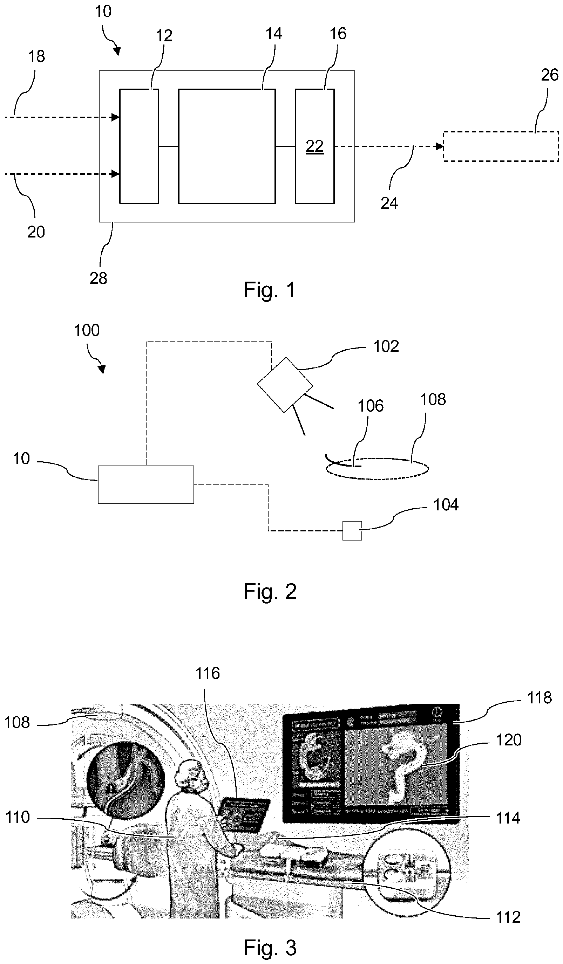

Fig. 1 schematically shows an example of a support device 10 for handling of an interventional device. The support device 10 comprises a data input 12, a data processor 14 and an output interface 16. The data input 12 is configured to provide 3D anatomic data, indicated with a first arrow 18, of a region of interest comprising a vascular structure, and to provide current spatial device data, indicated with a second arrows 20, of an interventional device inserted in the vascular structure. The data processor 14 is configured to analyze a mechanical relation 22 of the interventional device and the vascular structure based on the 3D anatomic data and the spatial device data. The output interface 16 is configured to provide the mechanical relation for a handling of the interventional device, as indicated with a third arrow 24.

A frame 26 in broken lines indicates a device for transmitting the feedback to the user, e.g. a tactile feedback device, as an option. Further, the data input 12, the data processor 14 and the output interface 16 can be arranged in a common housing 28. In an example, the data input 12, the data processor 14 and the output interface 16 are arranged separately, but data-connected.

The term "data input" relates to providing or supplying data for data processing steps. The data input 12 can also be referred to as image data input, e.g. when the data input 12 is mainly image data. The data input 12 can also be referred to as data supply, as image data supply, as image input, as input unit or simply as input. In an example, the data input 12 is data-connectable to an imaging source arrangement.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input 12 and the output interface 16.

The term "output interface" relates to an interface for providing the processed or computed data for further purposes. The output interface 16 can also be referred to as output or output unit. In an example, the output interface 16 is data-connectable to an output arrangement or output device for providing information or a signal to a user representing mechanical relation. In another example, the output interface 16 is data-connectable to a display arrangement or display device. As an example, the signals by the controller can be provided by the output interface 16.

In an example, the spatial device data is 2D image data. The term "spatial" relates to providing data that, in combination with other data sources like 3D data of the vascular structure, allows to retrieve the missing depth aspect of the 2D image data. Since the device is assumed to be arranged within the vessel structure, a 2D image of the device (arranged in the vessel structure) in combination with a 3D model or 3D image data of the vascular structure allows to add the depth information to the 2D image of the device.

In another example, the spatial device data is 3D image data of the device.

The term "support device" relates to a device that facilitates the operation of the interventional device and means e.g. better interaction for the user when using, i.e. operating the interventional device. The support device can also be referred to as support apparatus.

The term "handling of an interventional device" relates to using or operating the interventional device by a user. An example of operation is a manual operation by the user, e.g. a medical staff member like a physician or surgeon.

The term "interventional device" relates to any device configured for insertion into an anatomic structure of a subject. As an example, an interventional device is a guidewire, a catheter, an interventional tool, an imaging probe, a biopsy device, an examining tool or a therapeutic tool.

While the present application describes the interventional device being inserted in a vascular structure, also other devices are provided that are configured for movement within other body structure providing a lumen to maneuver within.

The term "3D anatomic data" relates to data representing the anatomy of the subject. The 3D anatomic data can be 3D imaging data of the subject, or a 3D model based on preoperational image data of the subject.

The term "current spatial device data" relates to data representing the interventional device as currently present within the anatomy.

The term "mechanical relation" relates to an interaction of the interventional device with the anatomy, e.g. the vascular structure. The mechanical relation relates to e.g. friction of the device inside the vascular structure, or tension built-up within the device, or blood flow acting on the device, or interaction of a tool part engaging with an anatomic part.

The expression "provide the mechanical relation for a handling of the interventional device" relates to transferring the computed insights in a way to the user such that the handling of the interventional device is supported.

As an advantage, the user is provided with information about tension building up through friction against a vessel wall, and/or bending the device inside the arteries. The friction buildup in the device can be detected and, on release of the tension the tip of the device, sudden forward movements are prevented, thus avoiding accidental artery puncture.

In an option, not shown in detail, the analyzing of the mechanical relation comprises computing at least one of the group of: i) friction between the interventional device and the anatomy, and ii) tension within the interventional device. As an option, for the analyzing of the mechanical relation, data is provided comprising mechanical properties of the interventional device. As a further option, for analyzing of the mechanical relation, a tortuosity of the interventional device is determined.

The term "friction" relates to the friction between the device and the anatomy.

The term "tension" relates to forces acting within the device that can cause a deformation or any other change of the shape of the device.

Tension build up can also be derived from the deformation of the anatomy. For instance, when a catheter is inside a vessel, the tube-like form of the vessel may deform like a cylinder with a deformation.

In an option, not shown in detail, the output interface 16 is configured to provide the mechanical relation comprising the output of a feedback based on the mechanical relation to a user operating the interventional device. In an option, the output of the feedback comprises to generate at least one of the group of: haptic and/or tactile feedback, visual feedback, acoustic feedback and electricity feedback.

The term "haptic and/or tactile feedback" relates to feedback that the user can feel or sense via touch or contact, e.g. a vibrating or pressing or pulling interaction.

The term "visual feedback" relates to feedback that the user can see with his eyes, e.g. light changing its appearance, like a warning light. As another example, an alert is visually given via a user interface like a monitor or display.

The term "acoustic feedback" relates to feedback that the user can hear.

The term "electricity feedback" relates to feedback that the user can experience as acting on nerves providing a respective user reception.

In an option, the feedback is provided via at least one of the group of:
- wrist wearing device;
- finger wearing ring device; and
- arm or lower limb wearing device.

In an option, the feedback is provided to the user on a non-sterile side of the user, for example below a protective clothing.

In an example, a belt-wearing device is provided.

In an example, the finger ring wearing device provides a vibrational signal to an upper side of the finger.

According to an aspect, a medical staff is provided with tactile feedback, like with gloves that have tactile actuators at some or all fingertips, allowing him/her to feel alarms in the form of subtle tactile/vibrating feedback when a force build-up in a device like a wire or catheter is detected, also when no X-ray is used.

Besides the transfer of alarms, also other mechanical feedback of the used device and other types of feedback from an imaging system to the doctor are provided.

As an advantage, feedback is possible even when the user is not looking at screens or the like. This can prevent e.g. safety related sudden forward movements of devices inside the patient's body and can reduce the X-ray dose to the patient and doctor.

The field of use is rather broad. As an example, the provided solution can be applied for all kinds of interventions and examinations and operations where a longitudinal device is maneuvered within a lumen structure. An example of the field of use, but in no way limiting, is image guided therapy (IGT), but also robotic procedure automation (RPA). As an example, automation features are provided in combination with an image guided therapy imaging system. An option is the combination with a robotic device manipulation system.

In case processing regarding segmentation and/or quantification of the mechanical aspects of the anatomy and the interventional device are provided in the background, the results of this processing will be detectable on e.g. an imaging system or also in form of data provided to a robotic device manipulator or by the tactile feedback mechanism.

The present solution is in particular helpful when applying thinner guidewires and catheters, when friction or other mechanical interaction cannot be felt through the thin wires on the fingertips. The present solution introduces an explicit tactile feedback channel next to visual cues on the imaging system. As an option, tangible feedback of stress/tension on the device is provided, e.g. directly towards the doctors' fingertips.

The examples described herein can provide a combination of feedback from measured mechanical stresses of devices in the subjects' arteries with visual and tangible cues of the imaging system to alert the physician timely of force build-up in the devices that could lead to unexpected movements of these devices.

As an advantage, the user is provided with information that reduces the need to map a manipulation of the device like a wire or catheter with the movement of the tip of the device inside the arteries of a patient. The so-called mental mapping of (finger) movement and the resulting movement of the device on the image is reduced and even less required, since the information thus retrieved is now provided as supplemental information, such as by a feedback device.

The device can make bends, touch the vessel wall or can pass through narrow parts, and the user is provided with respective feedback. Even with longer distances that the device travels inside a vessel, which may lead to larger tension, the user is provided with feedback that allows a better control or application of a pushing force. The user can thus focus on the tip of the device.

The present solution provides assisting a user by e.g. detecting tension on a visual image, and as such enhancing what the doctor could have been able to detect him/herself. In an example, the information of increased tension is routed to the user in a way that the user can receive and process that information, especially in times of high mental load. In an option, the tension between the fingers of the doctor and the device is measured and compared to 'normal values' keeping into account the length of the device, and the shape of its path through the patients' vessels.

In an example, a tip travel distance information can be measured from an image and compared with the travel distance at the entry point. This way, the user, e.g. a physician, could be warned if this relation is not 1:1 anymore and the device might be bending or buckling, also referred to as `curling up', somewhere out of view. This means that the physician is warned in case of unfavorable things happening outside of his view.

In an option, not shown in detail, to provide the 3D anatomic data, the data input 12 is configured to provide image data of the region of interest of the anatomy of the subject with the inserted interventional device. The data processor 14 is configured to determine the vascular structure in the region of interest accommodating at least a part of the interventional device.

In an option, not shown in detail, to determine the vascular structure, the data processor 14 is configured to provide anatomical modelling. As an option, for the anatomical modelling, the data processor 14 is configured to generate a 3D vessel model representing the vascular structure. As another option, to generate the 3D vessel model, the data processor 14 is configured to segment the vascular structure in the provided image data, and to quantify the vascular structure in its dimensions.

The term "anatomical modelling" relates to computing steps that result in a model of the anatomy such that a model of the device can be registered in order to perform further computational steps.

In an option, not shown in detail, to provide the 3D anatomic data, the data input 12 is configured to provide a 3D model of the region of interest of the anatomy of the subject. The data processor 14 is configured to register the 3D model to the current spatial device data.

In an option, to provide the 3D anatomic data, the data input 12 is configured to provide a 3D model of the region of interest of the anatomy of the subject based on pre-operative image data, e.g. image data acquired before the current intervention or operation. The 3D model may be adapted based on a current arrangement of the inserted interventional device.

In an option, not shown in detail, to provide the spatial device data, the data processor 14 is configured to identify and extract the interventional device within the vascular structure.

In an option, not shown in detail, to identify and extract the interventional device, the data processor 14 is configured to provided mechanical modelling. As an option, for the mechanical modelling, the data processor 14 is configured to generate a 3D device model representing the interventional device. In another option, to generate the 3D device model, the data processor 14 is configured to segment the interventional device in the provided image data, and to quantify the interventional device in its dimensions.

The term "mechanical modelling" relates to computing steps that result in a model of the device such that a model of the anatomy can be registered in order to perform further computational steps.

In an option, not shown in detail, to provide the spatial device data, the data input 12 is configured to provide shape data from shape sensing provided by the interventional device. The term "shape data" relates to data representing the current shape or allowing to compute the current shape.

For example, fiber optic real shape sensing (FORS) is provided. As an example, an optic shape sensing guidewire is provided that has an integrated optical fiber. By pulsing light into the fiber and analyzing how it is reflected, the full shape of devices can be reconstructed and visualized. In an option, such device is shown in the context of the patient's anatomy through overlay on images that are acquired before or during the procedure, like CT scans and X-rays.

In an option, not shown in detail, the data input 12 is configured to provide image data as a sequence of images. The data processor 14 is configured to provide the image analysis steps comprising at least the extracting of the interventional device as video-processing for a plurality of the images.

The term "sequence of images" relates to a plurality of images, e.g. a stream of images like a video stream.

In an example, the anatomical modelling also comprises computing a blood flow within the vascular structure.

In an example, the vascular structure in the provided image data is segmented and the vascular structure is quantified in its dimensions and an anatomic model is generated.

In another example, a 3D model is provided and registered to the provided image data.

As a first example, a generic 3D model of an anatomy is provided and adapted to the current determined vascular structure.

As a second example, a subject 3D model of the subject's is generated based on pre-interventional image data and the subject 3D model is adapted to the current determined vascular structure.

In an option, not shown in detail, the data input 12 is configured to provide additional sensor data relating to a current mechanical relation. The data processor 14 is configured to compare the additional sensor data with the analyzed mechanical relation to determine discrepancy information. The output interface 16 is configured to provide the discrepancy information for an adjustment.

The term "additional sensor data" relates to further data, like pressure data of a pressure sensor measuring the intensity of a force of the device against a portion of the anatomic structure.

The term "discrepancy information" relates to a deviation or mismatch of the computed data and the additionally measured data.

In an example, the discrepancy information is used for a plausibility check of an outcome of the analyzing of the mechanical relation.

As an example, the additional sensor data is provided by at least one additional sensor and the discrepancy information is used as calibration for the at least one additional sensor. In other words, an adjustment of the at least one additional sensor is provided.

As another example, the discrepancy information is used as calibration for the analyzing of the mechanical relation.

In an example, a force sensor is provided for determining a user's handling force acting on the device. For example, a glove with at least one force sensor is provided.

In an example, the force sensor is a pressure sensor that is provided for determining a user's handling force acting on the device. For example, a glove with at least one pressure sensor is provided.

In an option, comparison of the measured valued of the tension with friction that can be regarded as 'normal' could be an artificial intelligence model in advanced solutions, potentially taking the annotated anatomy and device shape and mechanical aspects into account, or a lookup table in basic solutions.

In an option, algorithms that run on a processor that compare measured friction against normal values can be based on a deep learning network such as a neural network.

Fig. 2 shows an example of a system 100 for handling of an interventional device inserted into a subject. The system 100 comprises a device 102 for providing 3D anatomic data of the subject. The system 100 further comprises an example of the support device 10 according to one of the preceding examples. The system 100 further comprises a feedback device 104. The device 102 for providing 3D anatomic data of the subject provides 3D anatomic data of a region of interest comprising a vascular structure to the support device 10. The feedback device 104 is data connected to the support device 10 and is configured to output a feedback to the user based on the mechanical relation.

It is noted that the device 102 for providing the 3D anatomic data is shown with a camera-like symbol. However, as also explained below, different types of data provision arrangements are provided. An example are medical imaging systems, like X-ray, MRI, CT, and ultrasound systems. Another example is stored anatomic data of the subject.

In an option, the system 100 also comprises an interventional device 106, for example chosen from a plurality of interventional devices.

In Fig. 2, an object 108 is schematically indicated, into which the interventional device 106 can be inserted to be navigated within.

In an option, the system 100 itself does not comprise an interventional device, but the user handles any interventional device.

In an option, the device 102 for providing 3D anatomic data of the subject is an imaging device 108, as indicated in Fig. 3. In the example shown, the imaging device 108 is configured to provide X-ray imaging data. The example shows a part of a C-arm imaging system. In a further option, an X-ray CT scanner is provided.

As a further option, although not shown in detail, the imaging device 108 is configured to provide magnetic resonance imaging data.

As a still further option, although not shown in detail, the imaging device 108 is configured to provide ultrasound imaging data.

Fig. 3 shows an illustration of another example of the system 100 for handling of an interventional device inserted into a subject. A medical staff member 110, e.g. a surgeon, is shown standing next to a subject support 112, on which a subject 114 is arranged, e.g. under sterile draping. The staff member 110 operates an interface 116, e.g. for handling an interventional device (not shown). A display 118 provides information about the current situation, e.g. presenting an indication of the status of the imaging system 108 in the left part of the screen and images of the region of interest in the right part showing anatomic details 120, e.g. with an inserted device currently operated by the staff member 110.

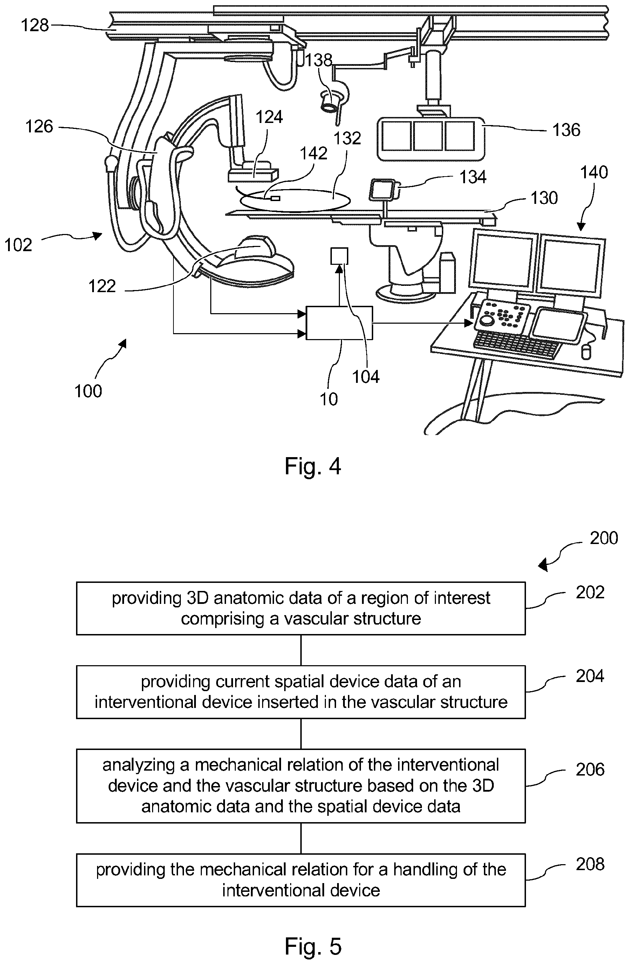

Fig. 4 shows an illustration of a further example of the system 100 for handling of an interventional device inserted into a subject. The device 102 for providing 3D anatomic data of the subject is provided as an X-ray imaging system with an X-ray source 122 and an X-ray detector 124 mounted to a C-arm structure 126. The C-arm structure is movably mounted to a ceiling rail support 128. A so-called patient table 130 is provided as support for a subject 132. Further, a side controller 134 is provided. Further, a display arrangement 136 is shown, together with movable lighting devices 138. A console 140 is shown in the right foreground, providing interfaces like a keyboard, mouse, graphic tablet, displays and control knobs.

An example of the support device 10 is schematically shown between the X-ray imaging system, the patient table 130 and the console 140. However, the support device 10 can also be arranged integrated into one of the shown equipment parts. The support device 10 is data-connected to the X-ray imaging system for receiving image data for the 3D anatomic data. Further, the support device 10 is also data-connected to the X-ray imaging system for receiving data for the current spatial device data. However, the support device 10 can also be connected for other data sources for the current spatial device data and the 3D anatomic data.

Still further, a device 142 like an interventional device is partly inserted into the subjected and navigated therein, e.g. by a physician. The feedback device 104 provides support for the user when handling the device 142. As an example, the feedback device 104 is a tactile device. In an option, the feedback device 104 is worn by a user, e.g. below his protective clothing.

In an option, the imaging system is separated from the processor unit and the feedback system. This allows for real-time (in-vivo), but also for non-realtime implementation such as training environments.

In an example, a system is provided comprising an imaging system, producing images of the anatomy (vessels) of the body, and an interventional device, inserted into these vessels, a tactile feedback system attached to the hands of the physician and/or another output receiving system, and a processor capable of analyzing images and determining the shape of the anatomy (vessel segmentation and quantification) and the device (segmentation and quantification) traveling through it and determining the friction and tension between the anatomy (vessel walls) and the interventional device and outputting the results to the tactile feedback system.

In an example, a system is provided comprising an imaging system, capable of receiving feedback from a processor on friction buildup, an interventional device, inserted into these vessels, a measuring system attached to the hands of the physician capable of measuring the force that is needed to push the interventional device forward, and a processor capable of analyzing the force on the interventional device and comparing this to normal values and by reaching a certain threshold, outputting a warning to the imaging system.

In an example, a system is provided comprising an imaging system, capable of receiving feedback from a processor on friction buildup, an interventional device, inserted into these vessels, a robotic manipulation system for interventional devices, a measuring system attached to the controllers of the robotic manipulation system, capable of measuring the force that is needed to push the interventional device forward, and a processor capable of analyzing the force on the interventional device and comparing this to normal values and by reaching a certain threshold, outputting a warning to the imaging system and stopping the robotic manipulation of the interventional device.

In an example, a system is provided comprising an imaging system, producing images of the anatomy (vessels) of the body, and an interventional device, inserted into these vessels. The interventional device is equipped with fiber optics real shape sensing (FORS) capable of measuring also the force exerted to the vessel wall. The system further comprises a tactile feedback system attached to the hands of the physician and/or another output receiving system, and a processor capable of analyzing images and determining the shape of the anatomy (vessel segmentation and quantification) and the device (segmentation and quantification) traveling through it. The processor is also capable of determining the friction and tension between the anatomy (vessel walls) and the interventional device based on image processor and/or FORS sensing and outputting the results to the tactile feedback system.

In an example, a system is provided comprising an imaging system, producing images of the anatomy (vessels) of the body, an interventional device, inserted into these vessels, a tactile feedback system attached to the hands of the physician and/or another output receiving system, and a processor capable of analyzing images and determining the device tip movement as well as sensor, e.g. a camera or other sensor, measuring the travel distance of the device into the patient. The processor is also capable of comparing the inserted device distance and the moved distance of the device tip and outputting the results to the tactile feedback system.

Fig. 5 shows basic steps of an example of a method 200 for supporting a handling of an interventional device. The method 200 comprises the following steps:
- In a first step 202, 3D anatomic data of a region of interest comprising a vascular structure is provided.
- In a second step 204, current spatial device data of an interventional device inserted in the vascular structure is provided.
- In a third step 206, a mechanical relation of the interventional device and the vascular structure is analyzed based on the 3D anatomic data and the spatial device data.
- In a fourth step 208, the mechanical relation for a handling of the interventional device is provided.

In an example, the first step takes place before the second step. The first and the second step can also be arranged in another order, e.g. the first and the second step can take place at the same time, or the second step takes place before the first step.

In an example, processing is done in a two-step approach. In a first step, the anatomy and the used device are detected, segmented and quantified. Also the positions where they touch each other, the total area/length of interaction and in which curvature is detected. In a second step, the interaction model between the vessel wall and the device can be filled with this information with a resulting output on force between the device and vessel wall. This output on force is translated to an interpretable warning on the chance of sudden release with a forward movement of the device. For example, a so-called stick-slip behavior of the device can be predicted and thus prevented or reduced in its effect.

When a meaningful threshold has been crossed, feedback can be transmitted to the user via a tangible feedback mechanism in the form of wearable gloves. The advantage of gloves is that it is non-intrusive, localized and is seen as a natural sensing and feedback position as the wire manipulation is normally done with the fingers. In an example, it enhances the doctors' skills with tactile feedback of the mechanical behavior of the used devices.

In another example, an imaging system is capable of receiving feedback from a processor on friction buildup. The interventional device is inserted into the vessels of the body. A measuring system attached to the hands of the physician is capable of measuring the force that is needed to push the interventional device forward. A processor analyzes the force on the interventional device and compares this to normal values and by reaching a certain threshold, a warning is outputted to the imaging system.

In another example, an imaging system is capable of receiving feedback from a processor on friction buildup. The interventional device is inserted into the vessels and a robotic manipulation system for interventional devices is provided. A measuring system is attached to the controllers of the robotic manipulation system and measures the force that is needed to push the interventional device forward. A processor analyzes the force on the interventional device and compares this to normal values and when reaching a certain threshold, a warning is outputted to the imaging system and the robotic manipulation of the interventional device is stopped.

In an example of the method, the analyzing of the mechanical relation comprises computing friction between the interventional device and the anatomy, and/or computing tension within the interventional device. As an option, for the analyzing of the mechanical relation, data is provided comprising mechanical properties of the interventional device. As a further option, for analyzing of the mechanical relation, determining of a tortuosity of the interventional device is provided.

In an example of the method, the providing of the 3D anatomic data comprises providing image data of the region of interest of the anatomy of the subject with the inserted interventional device; and determining the vascular structure in the region of interest accommodating at least a part of the interventional device.

In an example of the method, the determining of the vascular structure comprises anatomical modelling. As an option, the anatomical modelling comprises generating a 3D vessel model representing the vascular structure. As another option, for generating the 3D vessel model, it is provided segmenting the vascular structure in the provided image data; and quantifying the vascular structure in its dimensions.

In an example, the anatomical modelling also comprises computing a blood flow within the vascular structure.

In an example of the method, the providing of the 3D anatomic data comprises providing a 3D model of the region of interest of the anatomy of the subject; and registering the 3D model to the current spatial device data.

In an example of the method, the providing of the spatial device data comprises identifying and extracting the interventional device within the vascular structure.

In an example of the method, the identifying and extracting of the interventional device comprises mechanical modelling. As an option, the mechanical modelling comprises generating a 3D device model representing the interventional device. As another option, for generating the 3D device model, it is provided segmenting the interventional device in the provided image data and quantifying the interventional device in its dimensions.

In an example of the method, the providing of the spatial device data comprises providing shape data from shape sensing provided by the interventional device.

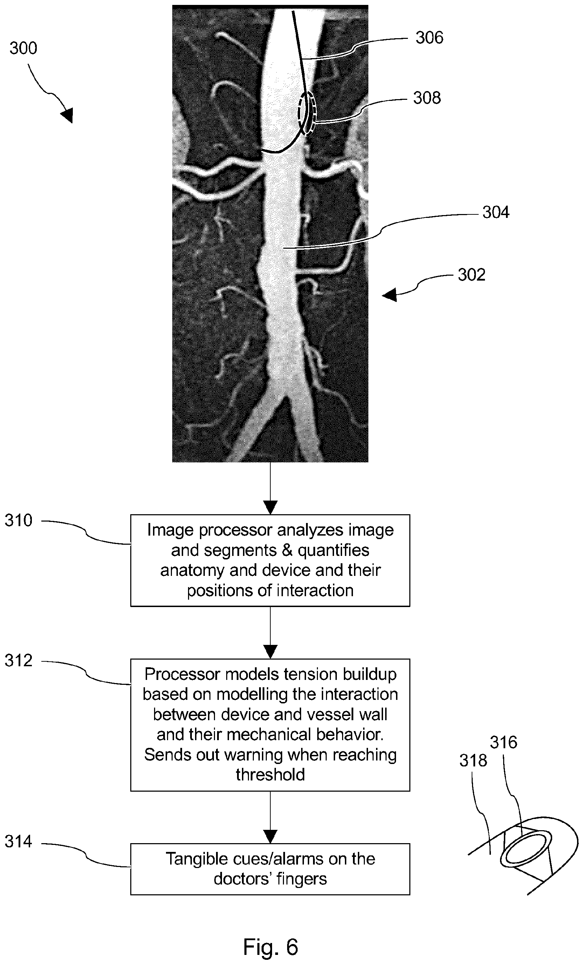

Fig. 6 shows a working scheme 300 of a first example. An X-ray image 302 provides anatomic information. The X-ray image 302 shows a vascular structure 304 and an inserted device 306, e.g. a catheter or guidewire. A location where the device 306 touches the vessel walls is highlighted with a broken-line indicator 308. A first frame 310 indicates that an image processor analyzes the image and segments and quantifies the anatomy and the device and their positions of mutual interaction. A second frame 312 indicates that the processor models a tension buildup based on modelling the interaction between the device and the vessel wall and their mechanical behavior. A warning can be provided when reaching a threshold. A third frame 314 indicates to provide tangible cues or alarms, e.g. on the physician's finger.

An illustration on the right indicates such a feedback device in form of a small tactile apparatus 316 worn on a fingertip 318.

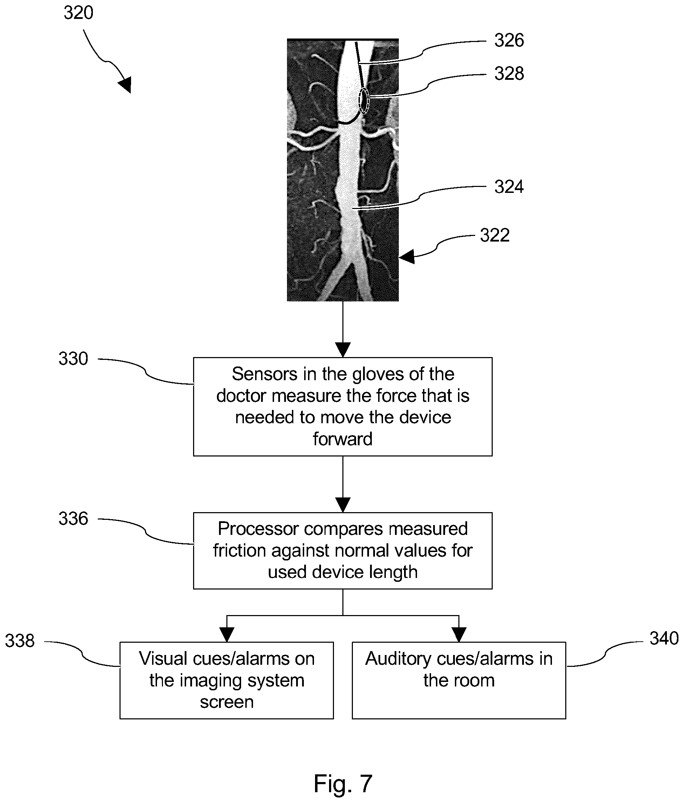

Fig. 7 shows a working scheme 320 of a second example with a variation. An X-ray image 322 provides anatomic information. The X-ray image 322 shows a vascular structure 324 and an inserted device 326, e.g. a catheter or guidewire. A location where the device 326 touches the vessel walls is again highlighted with a broken-line indicator 328. A first frame 330 indicates that sensors in a glove of a user measure the force that is needed to move the device forward. Such a glove may be provided with a plurality of force sensors arranged on surfaces of the glove that are used for contacting devices and equipment. A second frame 336 indicates that a processor compares measured friction against normal values for the used device length. As a first output, a third frame 338 provides visual cues or alarms on the imaging system screen. A fourth frame 340 indicates the output of auditory cues or alarms in the room. In other words, the mechanical relation in this example is determined and provided by dedicated glove sensors instead of image analysis, although a combination of both is also possible.

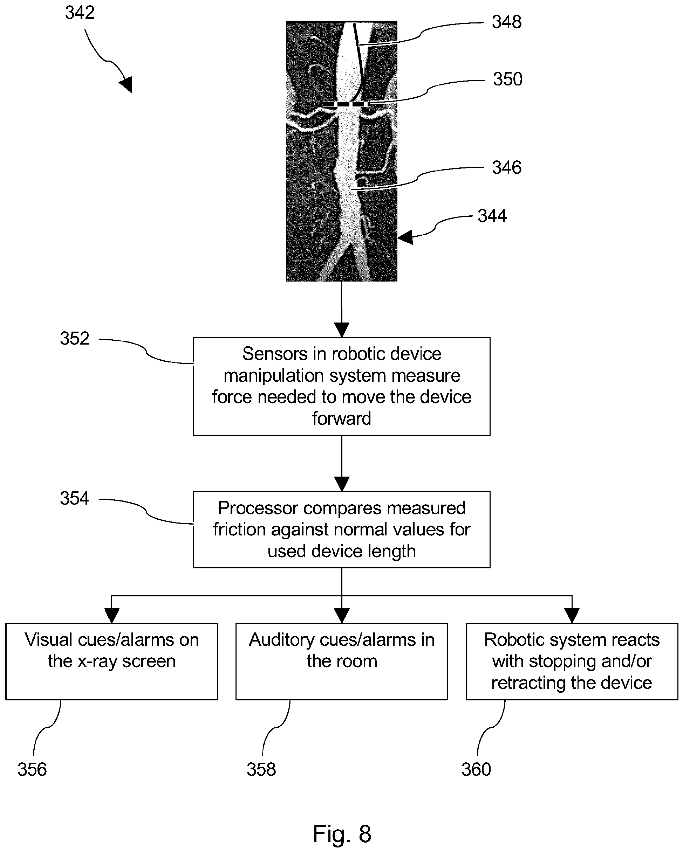

Fig. 8 shows a further working scheme 342 of a third example with a further variation. An X-ray image 344 provides anatomic information. The X-ray image 344 shows a vascular structure 346 and an inserted device 348, e.g. a catheter or guidewire. A location where the device 348 comes to a halt due to touching the vessel walls is highlighted with a broken-line indicator 350. A first frame 352 indicates that sensors in a robotic device manipulation system measure force needed to move the device forward. A second frame 354 indicates that a processor compares measured friction against normal values for the used device length. As a first output, a third frame 356 provides visual cues or alarms on the X-ray imaging system screen. A fourth frame 358 indicates the output of auditory cues or alarms in the room. A fifth frame 360 indicates that the robotic system reacts with stopping and/or with retracting the device. In other words, the mechanical relation in this example is determined and provided by dedicated robotic sensors instead of image analysis, although a combination of both is also possible.

The term "subject" may also be referred to as individual. The "subject" may further also be referred to as patient, although it is noted that this term does not indicate whether any illness or disease is actually present with the subject.

In an example, a computer program is provided that comprises instructions which, when the program is executed by a computer, cause the computer to carry out the method of one of the examples above.

In another exemplary embodiment, a computer program or a computer program element is provided that is characterized by being adapted to execute the method steps of the method according to one of the preceding embodiments, on an appropriate system.

In an example, a computer readable medium is provided having stored the computer program of the previous example.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g. microcode) to perform the required functions. A controller may however be implemented with or without employing a processor, and also may be implemented as a combination of dedicated hardware to perform some functions and a processor (e.g. one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A support device (10) for handling of an interventional device, the support device comprising:
- a data input (12);
- a data processor (14); and
- an output interface (16);
wherein the data input is configured to provide 3D anatomic data (18) of a region of interest comprising a vascular structure, and to provide current spatial device data (20) of an interventional device inserted in the vascular structure;
wherein the data processor is configured to analyze a mechanical relation (22) of the interventional device and the vascular structure based on the 3D anatomic data and the spatial device data; and
wherein the output interface is configured to provide the mechanical relation for a handling of the interventional device.

2. Support device according to claim 1, wherein the analyzing of the mechanical relation comprises computing at least one of the group of:
i) friction between the interventional device and the anatomy, and
ii) tension within the interventional device;
wherein, for the analyzing of the mechanical relation, data is provided comprising mechanical properties of the interventional device; and
wherein, for analyzing of the mechanical relation, a tortuosity of the interventional device is determined.

3. Support device according to claim 1 or 2, wherein the output interface is configured to provide the mechanical relation comprising to output a feedback based on the mechanical relation to a user operating the interventional device; and
wherein, the output of the feedback comprises to generate at least one of the group of:
- haptic and/or tactile feedback;
- visual feedback;
- acoustic feedback; and
- electricity feedback.

4. Support device according to claim 1, 2 or 3, wherein, to provide the 3D anatomic data, the data input is configured to provide image data of the region of interest of the anatomy of the subject with the inserted interventional device; and
the data processor is configured to determine the vascular structure in the region of interest accommodating at least a part of the interventional device.

5. Support device according to claim 4, wherein, to determine the vascular structure, the data processor is configured to provide anatomical modelling;
wherein, for the anatomical modelling, the data processor is configured to generate a 3D vessel model representing the vascular structure; and
wherein, to generate the 3D vessel model, the data processor is configured to segment the vascular structure in the provided image data, and to quantify the vascular structure in its dimensions.

6. Support device according to claim 1, 2 or 3, wherein, to provide the 3D anatomic data, the data input is configured to provide a 3D model of the region of interest of the anatomy of the subject, and the data processor is configured to register the 3D model to the current spatial device data.

7. Support device according to one of the claims 1 to 6, wherein, to provide the spatial device data, the data processor is configured to identify and extract the interventional device within the vascular structure.

8. Support device according to claim 7, wherein, to identify and extract the interventional device, the data processor is configured to provide mechanical modelling;
wherein for the mechanical modelling, the data processor is configured to generate a 3D device model representing the interventional device; and
wherein to generate the 3D device model, the data processor is configured to segment the interventional device in the provided image data, and to quantify the interventional device in its dimensions.

9. Support device according to one of the claims 1 to 6, wherein, to provide the spatial device data, the data input is configured to provide shape data from shape sensing provided by the interventional device.

10. Support device according to one of the claims 4 to 9, wherein the data input is configured to provide image data as a sequence of images; and
wherein the data processor is configured to provide the image analysis steps comprising at least the extracting of the interventional device as video-processing for a plurality of the images.

11. Support device according to one of the preceding claims, wherein the data input is configured to provide additional sensor data relating to a current mechanical relation;
wherein the data processor is configured to compare the additional sensor data with the analyzed mechanical relation to determine discrepancy information; and
wherein the output interface is configured to provide the discrepancy information for an adjustment.

12. A system (100) for handling of an interventional device inserted into a subject, the system comprising:
- a device (102) for providing 3D anatomic data of the subject;
- a support device (10) according to one of the preceding claims; and
- a feedback device (104);
wherein the device for providing 3D anatomic data of the subject provides 3D anatomic data of a region of interest comprising a vascular structure to the support device; and
wherein the feedback device is data connected to the support device and is configured to output a feedback to the user based on the mechanical relation.

13. System according to claim 12, wherein the device for providing 3D anatomic data of the subject is an imaging device (106); and
wherein the imaging device is configured to provide image data of at least one of the group of
- X-ray imaging;
- magnetic resonance imaging; and
- ultrasound imaging.

14. A method (200) for supporting a handling of an interventional device, the method comprising the following steps:
- providing (202) 3D anatomic data of a region of interest comprising a vascular structure;
- providing (204) current spatial device data of an interventional device inserted in the vascular structure;
- analyzing (206) a mechanical relation of the interventional device and the vascular structure based on the 3D anatomic data and the spatial device data; and
- providing (208) the mechanical relation for a handling of the interventional device.

15. Computer program comprising instructions which, when the program is executed by a computer, cause the computer to carry out the method of claim 14.
